# EUROPEAN PATENT SPECIFICATION

(11) **EP 1 809 342 B1**
(45) Date of publication and mention of the grant of the patent: **05.08.2015**
(21) Application number: 05813108.7
(22) Date of filing: 17.10.2005
(51) Int. Cl.: A61L 15/32, A61L 15/42, A61L 15/64

(54) **ABSORBABLE HEMOSTAT**
ABSORBIERBARE ARTERIENKLEMME
HEMOSTATIQUE ABSORBABLE

(30) Priority: 20.10.2004 US 620539 P; 01.07.2005 US 696258 P
(43) Date of publication of application: 25.07.2007
(73) Proprietor: Ethicon, Inc., Somerville, NJ 08876-0151 (US); Omrix Biopharmaceuticals, Inc., 1640 Rhode-st-Genese (BE)
(72) Inventor: ZHANG, Guanghui, Belle Mead, NJ 08502 (US); MARTINS, Sonia, Elizabeth, NJ 07202 (US); BARONE, Robin, Flemington, NJ 08822 (US)
(74) Representative: Kirsch, Susan Edith
(86) International application number: PCT/US2005/037403
(87) International publication number: WO 2006/044879

(56) References cited:
- WO-A-95/12371
- US-A- 6 054 122
- US-A1- 2004 005 350

## Description

The present invention relates to a hemostat.

The control of bleeding, as well as sealing of air and various bodily fluids, is essential and critical in surgical procedures to minimize blood loss, to seal tissue and organ structures, to reduce post-surgical complications, and to shorten the duration of the surgery in the operating room.

In an effort to provide dressings with enhanced hemostatic and tissue sealing and adhering properties, therapeutic agents, including, but not limited to, thrombin, fibrin and fibrinogen have been combined with dressing carriers or substrates, including gelatin-based carriers, polysaccharide-based carriers, glycolic acid or lactic acid-based carriers and a collagen matrix. Examples of such dressings are disclosed in US-B-6,762,336, US-B-6,733,774 and WO-A-2004064878. Fibrin sealant dressings are described in detail in US-B-6054122.

WO-A-9512371 describes a hemostatic patch comprising a biodegradable matrix such as an absorbable gelating sponge, and an effective amount of epsilon aminocaproic acid for inhibiting fibrinolysis. The patch can further contain thrombin or other active agents.

Due to its biodegradability and its bactericidal, tissue sealing, tissue repairing, drug delivering and hemostatic properties, it is desirable to utilize cellulose that has been oxidized to contain carboxylic acid moieties, hereinafter referred to as carboxylic-oxidized cellulose, as a topical dressing in a variety of surgical procedures, including neurosurgery, abdominal surgery, cardiovascular surgery, thoracic surgery, head and neck surgery, pelvic surgery and skin and subcutaneous tissue procedures.

However, when carboxylic-oxidized cellulose is utilized in combination with thrombin and fibrinogen, the acidic moieties that may be present in the cellulose denature the activity of the thrombin and fibrinogen. Therefore, it is desirable to shield the thrombin and fibrinogen from such acid moieties to maintain their hemostatic activities. US-A-20040005350 describes hemostatic wound dressings comprising an oxidized cellulose fabric and a porous, polymeric matrix of a biocompatible, water-soluble polymer substantially distributed through the fabric. The dressings may comprise additional hemostatic agents such as fibrinogen or thrombin.

The present invention provides a hemostat comprising: an absorbable foam of a biocompatible water-soluble or water-swellable polymer and a foaming agent/surfactant; an absorbable woven or knitted reinforcement fabric comprising carboxyl-oxidized cellulose, wherein the reinforcement fabric provides a backing to which the absorbable foam is directly or indirectly attached; and thrombin and fibrinogen, wherein the absorbable foam serves as a carrier for the fibrinogen and thrombin and serves to shield the thrombin and fibrinogen from acidic moieties that are present in the reinforcement fabric.

The hemostat described herein provides and maintains effective hemostasis when applied to a wound requiring hemostasis. Effective hemostasis, as used herein, is the ability to control and/or abate capillary, venous, or arteriole bleeding within an effective time, as recognized by those skilled in the art of hemostasis. Further indications of effective hemostasis may be provided by governmental regulatory standards and the like.

In certain embodiments, hemostats of the present invention are effective in providing and maintaining hemostasis in cases of severe or brisk bleeding. As used herein, severe bleeding is meant to include those cases of bleeding where a relatively high volume of blood is lost at a relatively high rate. Examples of severe bleeding include, without limitation, bleeding due to arterial puncture, liver resection, blunt liver trauma, blunt spleen trauma, aortic aneurysm, bleeding from patients with over- anticoagulation, or bleeding from patients with coagulopathies, such as hemophilia.

The thrombin and fibrinogen are substantially homogeneously dispersed throughout the foam and/or are disposed on the surface of the foam. The reinforcement fabric provides strength to the hemostat sufficient to permit the user to place and manipulate the hemostat on or within a wound or directly onto tissue of a patient requiring hemostasis, or tissue sealing and adhering.

In addition to serving as a carrier for the thrombin and fibrinogen, the foam also serves to shield the thrombin and fibrinogen from acidic moieties that may be present in the reinforcement fabric, such as is the case where carboxylic-oxidized cellulose is used as the reinforcement fabric.

The foam is a biocompatible, water-soluble, or water-swellable polymer and a foaming agent/surfactant. Preferred biocompatible, water-soluble, or water-swellable polymers used to fabricate the foam include polysaccharides. Such polysaccharides include, without limitation, cellulose, alkyl cellulose, e.g. methylcellulose, alkylhydroxyalkyl cellulose, hydroxyalkyl cellulose, cellulose sulfate, salts of carboxymethyl cellulose, carboxymethyl cellulose, and carboxyethyl cellulose. Additionally, albumin, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, chitosan, heparin, heparin sulfate, heparan, heparan sulfate, dermatan sulfate, keratan sulfate, carrageenans, chitosan, starch, amylose, amylopectin, poly-N-glucosamine, polymannuronic acid, polyglucuronic acid polyguluronic acid, and derivatives of any of the above, may be utilized. Even more preferably, biocompatible, water-soluble, or water-swellable polymers are an alkali or alkali earth metal salts of carboxylmethyl cellulose, most preferably sodium carboxylmethyl cellulose (CMC-Na).

The foaming agent/surfactant may be a cationic, anionic, amphoteric, zwitterionic or nonionic surfactant, or natural or modified proteins, including but without limitation, albumin, sodium lauryl sulfate, sodium laureth sulfate, or ammonia lauryl sulfate. A preferred foaming agent/surfactant is albumin, and more preferably, human serum albumin (HSA).

The reinforcement fabric is an absorbable woven or knitted fabric and comprises oxidized cellulose and the neutralized derivatives thereof. The cellulose is carboxylic-oxidized. More preferably, oxidized regenerated cellulose, may be used to prepare the second absorbable woven or knitted fabric. Regenerated cellulose is preferred due to its higher degree of uniformity versus cellulose that has not been regenerated. Regenerated cellulose and a detailed description of how to make oxidized regenerated cellulose are set forth in USP 3,364,200, USP 5,180,398 and USP 4,626,253.

Examples of fabrics that may be utilized as the reinforcement fabric include, but are not limited to, Interceed^{®} absorbable adhesion barrier, Surgicel^{®} absorbable hemostat, Surgicel Nu-Knit^{®} absorbable hemostat and Surgicel^{®} Fibrillar absorbable hemostat (each available from Johnson & Johnson Wound Management Worldwide or Gynecare Worldwide, each a division of Ethicon, Inc., Somerville, New Jersey).

The reinforcement fabric utilized in the present invention may be woven or knitted, provided that the fabric possesses the physical properties necessary for use in contemplated applications. Such fabrics, for example, are described in USP 4,626,253, USP 5,002,551 and USP 5,007,916. In preferred embodiments, the reinforcement fabric is a warp knitted tricot fabric constructed of bright rayon yarn that is subsequently oxidized to include carboxyl or aldehyde moieties in amounts effective to provide the fabrics with biodegradability.

In an alternative embodiment, the reinforcement fabric comprises oxidized polysaccharide fibers in combination with fibers comprised of aliphatic polyester polymers, copolymers, or blends thereof.

The reinforcement fabric preferably comprises oxidized regenerated cellulose and may have a basis weight ranging from 1.6 to 310 g/m² (0.001 to 0.2 g/in²), preferably in the range of 16 to 155 g/m² (0.01 to 0.1 g/in²), and most preferably in the range of 62 to 109 g/m² (0.04 to 0.07 g/in²).

In preparing the hemostats, a foam slurry may be prepared from, for example, CMC-Na and HSA, and then spread on the surface of, for example, an oxidized regenerated cellulose fabric. The ratio of the water-soluble or water-swellable polymer to the foaming agent may range from about 1:8 to 8:1 by weight, and preferably from about 2:1 to 1:2 by weight. The foam slurry is then dried either by lyophilization or in an oven at elevated temperature to form a solid foam substrate. The foam substrate may be treated with a chemical cross-linking agent such as glutaraldehyde for increased strength or may be partly cross-linked by heating. The density of the hemostat may be from about 5 to 20 mg/cm³.

The thrombin and fibrinogen may be animal derived, preferably human, or may be recombinant. The thrombin activity on the hemostat may be in the range of about 20 to 500 IU/cm², preferably about 20 to 200 IU/cm², and most preferably about 50 to 200 IU/cm². The fibrinogen activity on the hemostat may be in the range of about 2 to 15 mg/cm², preferably about 3 to 10 mg/cm², and most preferably about 4 to 7 mg/cm².

Thrombin powders may be prepared by lyophilization of thrombin solutions. Fibrinogen may be prepared by lyophilizaton of solutions containing fibrinogen, such as those described in USP 6,121,232 and PCT Application Publication No. WO 02/095019. Dry powders of fibrinogen and thrombin suspended in an organic solvent such as PF5060 or HFE 7000-7300 series are then sprayed onto the surface of the foam. Alternatively, the thrombin and fibrinogen may be incorporated into the foam during the foam production.

In an alternate embodiment, the hemostat may comprise a first foam having incorporated thereon or therein a first therapeutic agent, e.g., fibrinogen, and a second foam separate (unattached) from the first foam that may have upon or incorporated therein powders of a second therapeutic agent, e.g. thrombin. Alternatively, a hemostat may comprise a reinforcement fabric having a first foam adjacent thereto and second foam on the other side of the first foam. While either thrombin or fibrinogen may be incorporated into first foam, fibrinogen is preferred. While either thrombin or fibrinogen may be incorporated with the second foam, thrombin is preferred. In such an embodiment, the foam slurries used to prepare the first and second foams are selected such that the second foam is less dense than the first foam so that it liquefies or melts quickly after coming into contact with blood at the wound site to start the clotting process. The second foam contacts the bleeding site first, then the combined reinforcing layer and first foam keeps the clot from being washed away by the blood flow.

The hemostat may optionally include without limitation, procoagulant enzymes, proteins and peptides, may be naturally occurring, recombinant, or synthetic, and may be selected from the group consisting of prothrombin, fibrin, fibronectin, heparinase, Factor X/Xa, Factor VII/VIIa, Factor IX/IXa, Factor XI/XIa, Factor XII/XIIa, tissue factor, batroxobin, ancrod, ecarin, von Willebrand Factor, collagen, elastin, albumin, gelatin, platelet surface glycoproteins, vasopressin and vasopressin analogs, epinephrine, selectin, procoagulant venom, plasminogen activator inhibitor, platelet activating agents, synthetic peptides having hemostatic activity, derivatives of the above and any combination thereof.

The hemostat described herein may also be used as an adjunct to primary wound closure devices, such as arterial closure devices, staples, and sutures, to seal potential leaks of gasses, liquids, or solids as well as to provide hemostasis. For example, the hemostat may be utilized to seal air from tissue or fluids from organs and tissues, including but not limited to, bile, lymph, cerebrospinal fluids, gastrointestinal fluids, interstitial fluids and urine.

The hemostat described herein has additional medical applications and may be used for a variety of clinical functions, including but not limited to tissue reienforcement and buttressing, i.e., for gastrointestinal or vascular anastomoses, approximation, i.e., to connect anastomoses that are difficult to perform (i.e. under tension), and tension releasing. The hemostat may additionally promote and possibly enhance the natural tissue healing process in all the above events. This hemostat can be used internally in many types of surgery, including, but not limited to, cardiovascular, peripheral-vascular, cardio-thoracic, gynecological, neuro- and general surgery. The hemostat may also be used to attach medical devices (e.g. meshes, clips and films) to tissues, tissue to tissue, or medical device to medical device.

### Example 1. Lyophilized CMC-Na/ HSA foam with ORC fabric

In a mixing bowl were added 190 mL of a 2% solution of sodium carboxy methylcellulose (CMC-Na) (Aqualon, catalog No. 7M8SFPH) and 10 mL of a 20% solution of human serum albumin (HSA) (ALBUTEIN™, Alpha Therapeutic Corporation). The mixture was whipped mechanically to generate a foamed slurry. The foamed slurry was transferred to a rectangular frame having a piece of knitted carboxylic-oxidized regenerated cellulose fabric, available from Ethicon, Inc. under the tradename Interceed^{®}, disposed at the bottom. The foamed slurry was then spread evenly across the whole frame to yield a thickness of about 3 mm. The foamed slurry was then lyophilized to remove solvent, thus yielding a solid CMC-Na/HSA foam adjacent and attached to the ORC fabric.

### Example 2. Heat-treated CMC-Na/ HSA foam with ORC fabric

In a mixing bowl were added 190 mL of 2% solution of sodium carboxy methylcellulose (CMC-Na) (Aqualon, catalog No. 7M8SFPH) and 10 mL of 20% solution of human serum albumin (HSA) (ALBUTEIN™, Alpha Therapeutic Corporation). The mixture was whipped mechanically to generate a foamed slurry. The foamed slurry was transferred to a rectangular frame having a piece of carboxylic-oxidized regenerated cellulose fabric, available from Ethicon, Inc. under the tradename Interceed^{®}, at the bottom. The foamed slurry was then spread evenly across the whole frame to yield a thickness of about 3 mm. The foamed slurry was then heated in an oven at 65°C for an hour to remove solvent and thus yielded a solid CMC-Na/HSA foam adjacent and attached to the ORC fabric.

### Example 3. Reinforced Foams with active clotting factors

Hemostatic agents were applied to the constructs prepared in Examples 1 and 2 as below. The constructs were cut into 7.6 cm x 10.2 cm (3" x 4") pieces. Lyophilized thrombin and Biological Active Components 2 (BAC-2) containing fibrinogen were ground to powder separately. The thrombin powder and the BAC-2 powder were passed through a 45-micrometer sieve. The two powders were weighed to provide a final fibrinogen concentration of 6 mg/cm², 7 mg/cm² or 8 mg/cm², and a thrombin activity of 50 IU/cm². The powders were then mixed and suspended in a per-fluorinated solvent HFE 7000 in a flask. The suspension then was sprayed onto the various constructs and then dried under nitrogen at room temperature for 1 hour.

### Example 4. Hemostasis test in a swine spleen linear incision model

Linear incisions (1.5 cm long and 0.3 cm deep) were made on a swine spleen. After spraying the wound with 0.9% saline solution, various test materials from Example 3 were applied to the wounds. Tamponade was applied for 30 seconds followed by a 30-second observation. When hemostasis was not achieved, additional tamponade was applied to stop the bleeding. A piece of surgical gauze and a commercial product Tachocomb^{®} surgical patch (commercially available from Nycomed Pharma GmbH) were used as controls. Table 1 lists the experimental results.

**Table 1. Hemostasis test results in a swine spleen model.**

| Test material | Average number of tamponade | Average time to hemostasis (min:sec) |
|---|---|---|
| Surgical gauze | 21 | >12:00 |
| CMC-Na/HSA lyophilized foam/Interceed^{®} fabric backing 6 mg/cm² fibrinogen 50 IU/cm² thrombin | 4 | 2:33 |
| CMC-Na/HSA lyophilized foam/Interceed^{®} fabric backing 8 mg/cm² fibrinogen 50 IU/cm² thrombin | 3 | 2:13 |
| CMC-Na/HSA heat-treated foam/Interceed^{®} fabric backing 7 mg/cm² fibrinogen 50 IU/cm² thrombin | 2 | 1:03 |
| Tachocomb^{®} Surgical Patch (having 5.5 mg/cm² fibrinogen 2.0 IU/cm² thrombin) | 7 | 5:55 |

### Example 5. CMC-Na/HSA foam with thrombin

In a mixing bowl were added 190 mL of a 2% solution of sodium carboxy methylcellulose (CMC-Na) (Aqualon, catalog No. 7M8SFPH) and 10 mL of a 20% solution of human serum albumin (HSA) (ALBUTEIN™, Alpha Therapeutic Corporation). The mixture was whipped mechanically to generate a foamed slurry. A portion of the foamed slurry was transferred to a glass beaker. Thrombin powder was folded into the foamed slurry to yield an estimated activity of 1,000 IU/cm³. The final mixture was lyophilized in aluminum weighing dishes with a height of 0.5 cm to form a solid foam comprising the thrombin dispersed therethrough.

### Example 6. CMC-Na/HSA foam with fibrinogen

In a mixing bowl were added 190 mL of 2% solution of sodium carboxy methylcellulose (CMC-Na) (Aqualon, catalog No. 7M8SFPH) and 10 mL of 20% solution of human serum albumin (HSA) (ALBUTEIN™, Alpha Therapeutic Corporation). The mixture was whipped mechanically to generate a foamed slurry. A portion of the foamed slurry was transferred to a glass beaker. Five grams of fibrinogen powder was folded into the foam. The final mixture was placed in an aluminum weighing dish with a height of 0.5 cm and a having a piece of ORC fabric at the bottom. The foamed slurry then was lyophilized to form a solid foam comprising the fibrinogen dispersed therethrough.

### Example 7. Hemostasis test with CMC-Na/HSA/Thrombin foam and CMC-Na/HSA/Fibrinogen.foam

A severe bleeding wound was made on a swine liver. The defect was created by making a triangular cut with a surgical scalpel. Each side measured about 1 inch and the depth measured 5mm. After the triangular liver tissue was removed, CMC-Na/HSA/Thrombin foam 2.5 cm x 2.5 cm (1" x 1") was quickly applied to the wound. A piece of CMC-Na/HSA/Fibrinogen foam was then applied on top of the thrombin foam followed by manual compression. Hemostasis was achieved in 2 minutes.

## Claims

1. A hemostat comprising:
an absorbable foam of a biocompatible water-soluble or water-swellable polymer and a foaming agent/surfactant;
an absorbable woven or knitted reinforcement fabric comprising carboxylic-oxidized cellulose, wherein the reinforcement fabric provides a backing to which the absorbable foam is directly or indirectly attached; and
thrombin and fibrinogen, wherein the absorbable foam serves as a carrier for the fibrinogen and thrombin and serves to shield the thrombin and fibrinogen from acidic moieties that are present in the reinforcement fabric.

2. The hemostat of claim 1, where the foam comprises one or more polymers selected from the group consisting of polysaccharides, albumin, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, chitosan, heparin, heparin sulfate, heparan, heparan sulfate, dermatan sulfate, keratan sulfate, carrageenans, chitosan, starch, amylose, amylopectin, poly-N-glucosamine, polymannuronic acid, polyglucuronic acid polyguluronic acid, and derivatives of any of the above.

3. The hemostat of claim 2, where the foam comprises a polysaccharide selected from the group consisting of methylcellulose, alkylhydroxyalkyl cellulose, hydroxyalkyl cellulose, cellulose sulfate, salts of carboxymethyl cellulose, carboxymethyl cellulose, carboxyethyl cellulose.

4. The hemostat of any preceding claim, wherein the thrombin and fibrinogen are substantially uniformly dispersed throughout the foam and/or are dispersed on the surface of the foam

5. The hemostat of claim 1, wherein the haemostat comprises a first foam having incorporated therein fibrinogen and a second foam separate (unattached) from the first foam having, upon or incorporated therein, a thrombin powder.

6. The hemostat of claim 1, where the absorbable woven or knitted fabric comprises oxidized regenerated cellulose.

7. The hemostat of claim 6, where the absorbable woven or knitted fabric is an absorbable knitted fabric comprising oxidized regenerated cellulose.

8. The hemostat of claim 1, where the foam comprises carboxymethyl cellulose and albumin, the absorbable woven or knitted fabric comprises oxidized regenerated cellulose, and the thrombin and fibrinogen are incorporated into or sprayed onto the foam.

9. The hemostat of claim 8, where the weight ratio of carboxymethyl cellulose to the albumin ranges from 1:8 to 8:1 by weight.

10. The hemostat of claim 8, wherein the thrombin activity on the hemostat ranges from 20 to 500 IU/cm², and the fibrinogen activity ranges from 2 to 15 mg/cm².

## Patentansprüche

1. Arterienklemme, die Folgendes aufweist:
einen absorbierbaren Schaum eines biokompatiblen wasserlöslichen oder wasserquellfähigen Polymers und eines Schaumbildners/Tensids;
ein absorbierbares gewebtes oder gewirktes Verstärkungsgewebe, das carboxylisch oxidierte Cellulose aufweist, wobei das Verstärkungsgewebe eine Unterstützung bereitstellt, an der der absorbierbare Schaum direkt oder indirekt befestigt ist; und
Thrombin und Fibrinogen, wobei der absorbierbare Schaum als ein Träger für das Fibrinogen und das Thrombin dient und dazu dient, das Thrombin und das Fibrinogen von säurehaltigen Teilen abzuschirmen, die im Verstärkungsgewebe vorhanden sind.

2. Arterienklemme nach Anspruch 1, wobei der Schaum ein oder mehrere Polymere aufweist, die aus der Gruppe ausgewählt sind, die aus Polysacchariden, Albumin, Chitin, Carboxymethylchitin, Hyaluronsäure, Salze der Hyaluronsäure, Alginat, Alginsäure, Propylenglykolalginat, Glykogen, Dextran, Dextransulfat, Kurdlan, Pektin, Pullulan, Xanthan, Chondroitin, Chondroitinsulfaten, Carboxymethyldextran, Carboxymethylchitosan, Chitosan, Heparin, Heparinsulfat, Heparan, Heparansulfat, Dermatansulfat, Keratansulfat, Carrageene, Chitosan, Stärke, Amylose, Amylopektin, Poly-N-Glukosamin, Polymannuronsäure, Polyglucuronsäure, Polyguluronsäure und Derivate von irgendeinem des Obigen besteht.

3. Arterienklemme nach Anspruch 2, wobei der Schaum ein Polysaccharid aufweist, das aus der Gruppe ausgewählt ist, die aus Methylcellulose, Alkylhydroxyalkylcellulose, Hydroxyalkylcellulose, Cellulosesulfat, Salze der Carboxymethylcellulose, Carboxymethylcellulose und Carboxyethylcellulose besteht.

4. Arterienklemme nach einem der vorhergehenden Ansprüche, wobei das Thrombin und das Fibrinogen im Wesentlichen gleichmäßig im gesamten Schaum dispergiert sind und/oder auf der Oberfläche des Schaums dispergiert sind.

5. Arterienklemme nach Anspruch 1, wobei die Arterienklemme einen ersten Schaum, der darin eingebautes Fibrinogen aufweist, und einen zweiten Schaum aufweist, der vom ersten Schaum getrennt (nicht gebunden) ist, der darauf oder ein darin eingebautes Thrombin-Pulver aufweist.

6. Arterienklemme nach Anspruch 1, wobei das absorbierbare gewebte oder gewirkte Gewebe oxidierte regenerierte Cellulose aufweist.

7. Arterienklemme nach Anspruch 6, wobei das absorbierbare gewebte oder gewirkte Gewebe ein absorbierbares Gewirk ist, das oxidierte regenerierte Cellulose aufweist.

8. Arterienklemme nach Anspruch 1, wobei der Schaum Carboxymethylcellulose und Albumin aufweist, das absorbierbare gewebte oder gewirkte Gewebe oxidierte regenerierte Cellulose aufweist, und das Thrombin und das Fibrinogen in den Schaum eingebaut oder darauf gesprüht sind.

9. Arterienklemme nach Anspruch 8, wobei das Gewichtsverhältnis der Carboxymethylcellulose zu Albumin im Gewicht von 1:8 bis 8:1 reicht.

10. Arterienklemme nach Anspruch 8, wobei die Thrombin-Aktivität an der Arterienklemme von 20 bis 500 IU/cm² reicht, und die Fibrinogen-Aktivität von 2 bis 15 mg/cm² reicht.

## Revendications

1. Hémostatique comprenant :
une mousse absorbable d'un polymère biocompatible soluble dans l'eau ou gonflable à l'eau et d'un agent moussant/tensioactif ;
une toile de renforcement tissée ou tricotée absorbable comprenant de la cellulose carboxylique oxydée, la toile de renforcement fournissant une doublure à laquelle la mousse absorbable est directement ou indirectement attachée ; et
de la thrombine et du fibrinogène, la mousse absorbable servant de support pour le fibrinogène et la thrombine et servant à protéger la thrombine et le fibrinogène des fractions acides qui sont présentes dans la toile de renforcement.

2. Hémostatique selon la revendication 1, dans lequel la mousse comprend un ou plusieurs polymères choisis dans le groupe constitué par les polysaccharides, l'albumine, la chitine, la carboxyméthylchitine, l'acide hyaluronique, les sels d'acide hyaluronique, l'alginate, l'acide alginique, l'alginate de propylène glycol, le glycogène, le dextrane, le sulfate de dextrane, le curdlane, la pectine, le pullulane, le xanthane, la chondroïtine, les sulfates de chondroïtine, le carboxy-méthyldextrane, le carboxyméthylchitosane, le chitosane, l'héparine, le sulfate d'héparine, l'héparane, le sulfate d'héparane, le sulfate de dermatane, le sulfate de kératane, les carraghénanes, le chitosane, l'amidon, l'amylose, l'amylopectine, la poly-N-glucosamine, l'acide polymannuronique, l'acide polyglucuronique, l'acide polyguluronique, et les dérivés de tous les polymères précités.

3. Hémostatique selon la revendication 2, dans lequel la mousse comprend un polysaccharide choisi dans le groupe constitué par la méthylcellulose, une alkyl-hydroxyalkylcellulose, une hydroxyalkylcellulose, le sulfate de cellulose, les sels de carboxyméthylcellulose, la carboxyméthylcellulose, et la carboxyéthylcellulose.

4. Hémostatique selon une quelconque revendication précédente, dans lequel la thrombine et le fibrinogène sont dispersés de façon sensiblement uniforme dans toute la mousse et/ou sont dispersés sur la surface de la mousse.

5. Hémostatique selon la revendication 1, l'hémostatique comprenant une première mousse ayant, incorporé à l'intérieur, du fibrinogène et une deuxième mousse séparée (non attachée) de la première mousse ayant, par-dessus ou incorporée à l'intérieur, une poudre de thrombine.

6. Hémostatique selon la revendication 1, dans lequel la toile tissée ou tricotée absorbable comprend de la cellulose oxydée régénérée.

7. Hémostatique selon la revendication 6, dans lequel la toile tissée ou tricotée absorbable est un tricot absorbable comprenant de la cellulose oxydée régénérée.

8. Hémostatique selon la revendication 1, dans lequel la mousse comprend de la carboxyméthylcellulose et de l'albumine, la toile tissée ou tricotée absorbable comprend de la cellulose oxydée régénérée, et la thrombine et le fibrinogène sont incorporés dans ou pulvérisés sur la mousse.

9. Hémostatique selon la revendication 8, dans lequel le rapport pondéral de la carboxyméthylcellulose à l'albumine va de 1:8 à 8:1 en poids.

10. Hémostatique selon la revendication 8, l'activité de la thrombine sur l'hémostatique allant de 20 à 500 UI/cm², et l'activité du fibrinogène allant de 2 à 15 mg/cm².
